Europäisches Patentamt

European Patent Office

Office européen des brevets

(11) Publication number: **0 258 489**
A1

# EUROPEAN PATENT APPLICATION

(21) Application number: 86113073.0

(22) Date of filing: 23.09.86

(51) Int. Cl.³: **C 12 N 15/00**
C 07 K 15/06, C 12 P 21/00
C 12 Q 1/68, A 61 K 37/02

A request for correction of the description has been filed pursuant to Rule 88EPC. A decision on the request will be taken during the proceedings before the Examining Division.

(30) Priority: 21.08.86 EP 86111581

(43) Date of publication of application:
09.03.88 Bulletin 88/10

(84) Designated Contracting States:
GB

(71) Applicant: Kishimoto, Tadamitsu
Division of Cellular Immunology Institute of Molecular and Cellular Biology University Osaka
4-3-2, Nakoshima Kita-Ku-Osaka 530(JP)

(72) Inventor: Kishimoto, Tadamitsu, Prof. Dr.
3-5-31, Nakaro
Tondabayashi, Osaka 584(JP)

(72) Inventor: Suemura, Masaki, Dr.
9-5, Himemuro
Ikeda, Osaka 563(JP)

(72) Inventor: Kikutani, Hitoshi, Dr.
2-1-26, Esaka
Suita, Osaka 564(JP)

(72) Inventor: Barsumian, Edward L., Dr.
3-1-503, Senba-Nishi
Mino, Osaka 562(JP)

(74) Representative: Laudien, Dieter, Dr. et al,
Boehringer Ingelheim Zentrale GmbH ZA Patente
Postfach 200
D-6507 Ingelheim am Rhein(DE)

(54) Human low affinity FC epsilon-receptor, the isolation and purification thereof.

(57) The present invention is concerned with human low affinity Fc$_\varepsilon$-receptor and the water-soluble part thereof starting with amino acids from about 50 to about 150 of the human low affinity Fc$_\varepsilon$-receptor, preferably with the N-terminal Met-Glu-Leu-Gln-Val-Ser-Ser-Gly-Phe-Val-, their isolation and purification.

The prepared human low affinity Fc$_\varepsilon$-receptor, preferably the water-soluble part thereof, is suitable for the treatment of local and allergic reactions induced by expression of IgE and may be incorporated into the suitable pharmaceutical compositions.

EP 0 258 489 A1

# Human low affinity $Fc_\xi$-receptor, the isolation and purification thereof

Receptors for the Fc-portion of immunoglobuline (FcR) are expressed on various hematopoietic cell lineages and provide an important link between antibody molecules and their effector functions, such as internalization of the ligand-receptor complexes, antibody-mediated cytolysis of target cells and the release of inflammatory mediators. The expression of Fc-receptors has also been demonstrated on T and B lymphocytes, suggesting a possible role for FcR in the regulation of the immune response as well as the involvement of immunoglobulin (Ig)-binding factors, such as IgE-, IgA- and IgG-binding factors, in isotype specific regulation of the antibody response. At present, none of Fc-receptors or the genes encoding Fc-receptors have yet been isolated, whereby two kinds of Fc-receptors for IgE ($Fc_\xi R$) are known which differ in structure and function, namely

a) high affinity $Fc_\xi R$ on basophils and mast cells and
b) low affinity $Fc_\xi$-receptors on lymphocytes and monocytes.

$Fc_\xi$-receptor was found to be an insoluble membrane protein having the unusual and unexpected characteristic of an N-terminal in the cytoplasm and a C-terminal outside of the cell, contrary to known receptors. Moreover, an increase of water-soluble $Fc_\xi R$ as a complex with IgE was observed in the serum of atopic patients.

Therefore, this invention is concerned with human low affinity $Fc_\epsilon$-receptor and the water-soluble part thereof starting with amino acids from about 50 to about 150 of the whole $Fc_\epsilon$-receptor, preferably with the N-terminal Met-Glu-Leu-Gln-Val-Ser-Ser-Gly-Phe-Val-, their isolation and purification, whereby the following aspects are objects of the present invention:

a) Isolation and purification of the water-soluble part of IgE binding factor ($Fc_\epsilon R$) secreted or shed by lymphoblastoid cells;

b) Partial sequencing of the water-soluble part of the human low affinity $Fc_\epsilon$-receptor isolated according to a) by means of hydrolysis, isolating the thus obtained fragments and determination of the sequences of the thus obtained fragments;

c) Preparation of two hydridization probes:

Probe 1: Preparation of $Fc_\epsilon^+L$ cell transformant specific cDNA by using multiple cyclic substraction with $Ltk^-$ cell mRNA;

Probe 2: Preparation of an oligonucleotide encoding to one of the said partial sequences isolated according to b), preferably of a mixture of oligonucleotides of formula

$$3'-TT^T_C \ ACC \ TA^T_AG \ TT^A_G \ AA^A_G \ GT \ -5'$$

encoding for the amino acid sequence of formula

Lys-Trp-Ile-Asn-Phe-Gln;

d) Isolating and identifying of expression vehicles containing the gene coding for human low affinity $Fc_\varepsilon$-receptor, comprising the steps .

synthesizing cDNA from a RNA matrix derived from lymphoblastoid cells producing $Fc_\varepsilon$-receptor mRNA,

incorporating said synthesized cDNA in expression vehicles to form an expression vehicle bank,

hybridizing said incorporated cDNA to identify those expression vehicles which contain a gene coding for $Fc_\varepsilon$-receptor, with two labelled probes comprising cDNA specific to $Fc_\varepsilon R^+L$ cell and an oligonucleotide common to the gene of low affinity $Fc_\varepsilon$-receptor, and

replication of the thus obtained $Fc_\varepsilon$-receptor gene;

e) Expression of the $Fc_\varepsilon R$ cDNA;

f) Determination of the gene coding for the human low affinity $Fc_\varepsilon$-receptor utilising isolated cDNA sequence obtained from the vehicles from operation e) according to the sequencing methods of Sanger et al. and the chemical cleavage method of Maxam and Gilbert;

g) Expression of $Fc_\varepsilon R$ mRNA;

h) Preparation of an expression vector containing the DNA sequences coding for a water-soluble fragment of the $Fc_\varepsilon$-receptor and expressing said soluble fragment in microorganisms or in mammalian cells; and

i) Use of the expressed polypeptides for the treatment of local and systemic IgE-allergic reactions and the pharmaceutical compositions containing these polypeptides.

a) <u>Isolation and purification of water-soluble part of $Fc_\varepsilon R$</u>

Human B lymphoblastoid cells such as RPMI-8866 cells secret $Fc_\varepsilon R$ of about 46 kd on their surface and release a species of about 25 kd into the culture supernatent. Now, it was found, that the $Fc_\varepsilon R$ activity secreted or shed by lymphoblastoid cells, e.g. RPMI-8866 cells, as detected by the ELISA method (Figure 1) utilizing two different monoclonal antibodies (see European Patent Application No. 86 110 420.6 of the same applicant, filed on July 29, 1986) was higher in the concentrated culture supernatant compared to NP-40 detergent solubilized membrane receptors even though an equal number, e.g., $10^5$ cells/ml were utilized to prepare $Fc_\varepsilon R$. Furthermore, when affinitiy purified supernatants were chromatographed on SDS-PAGE under non-reducing conditions and $Fc_\varepsilon R$ activity eluted from portions of the gel corresponding to defined molecular weight, activity was observed (Figure 2) in the 45-46 kd and 24-25 kd regions. In fact the concentrated culture supernatants contained a higher proportion of activity in the 25 kd region. Therefore serum-free culture supernatants were used as the source of the receptor.

For the sequential immunoaffinity purification of $Fc_\varepsilon R$ with a molecular weight of about 25 kd were used immunoaffinity columns which were prepared utilizing 10 mg/ml of purified monoclonal antibody coupled to Sepharose 4B beads (Pharmacia, Piscataway, N.J.) as described by the manufacturer.

The sequential adsorption of 200-250 x concentrated culture supernatant on BSA-Sepharose, transferrin Sepharose and normal mouse IgG-Sepharose yielded about 70 percent of the original activity without however, improving on the specific activity. As shown in Table 1 following preadsorption the receptor material was allowed to bind to 3-5-Sepharose column for 4-16 hours, the total activity of the acetic acid eluate was reduced but the specific activity was increased

to 83 units/µg and the purification was 190 fold. Further purification of the receptor by HPLC reverse-phase chromatography on C-4 column using a linear gradient of 0-65 % acetonitril and 0,1 % trifluoracetic acid increased the specific activity to 1630 units/µg and the receptor was purified 3710 fold. However, the final recovery was only 33 percent of the original. As seen in Table 1, a similar purification scheme was used for detergent-solubilized membrane $Fc_\varepsilon R$ but the specific activity obtained was consistently lower than that observed with culture supernatants. It is important to note that the choice of elution buffer was critical to the level of recovery, 2.5 percent acetic acid elution with rapid neutralization was important in the final yield of the receptor.

As indicated in Table 1 immunoaffinity purification of $Fc_\varepsilon R$ utilizing the specific monoclonal antibodies in the solid phase was not sufficient to obtain pure receptor as measured by a single band on SDS-PAGE. Therefore, freshly eluted $Fc_\varepsilon R$ was further purified by means of HPLC Reverse Phase Purification. The freshly eluted $Fc_\varepsilon R$ was loaded preperatively on a C-4 column and chromatographed utilizing a linear gradient of 0-65 percent acetonitril the chromatographic profile is shown on Figure 3, fractions obtained at various retention times were tested by ELISA for $Fc_\varepsilon R$ activity. It was found that the bulk of the $Fc_\varepsilon R$ was eluted by acetonitril at between 44 and 45 percent concentration. When 0.5 ml samples were collected the $Fc_\varepsilon R$ activity corresponded to the descending slope of the peak indicated on the Figure 3. The rechromatographic analysis of the active fraction showed a single sharp peak indicating the presence of a homogenous mixture of receptor. The fractions obtained at different retention times were also monitored by SDS-PAGE analysis.

Therefore, the concentrated culture supernatant (crude sup) containing the putative $Fc_\varepsilon R$ and the material eluted from immunoaffinity and C-4 HPLC column were tested after dialysis and lyophilization on a 10 percent SDS-PAGE as described below. The results indicate the presence of multiple bands in the lane of the crude concentrated sup. A broad band corresponding to 22-24 kd can be seen.

The receptor moiety collected after sequential purification on non-specific and specific immunoaffinity gels still shows multiple bands even though the activity of such eluates is substantially higher than that of crude material. The $Fc_{\varepsilon}R$ activity (Figure 4) obtained after C-4 HPLC purification showed a single band corresponding to 25 kd and the material purified in this fashion showed very high activity in the ELISA assay utilizing the monoclonal antibodies. It is important to note that the band of 25 kd corresponds to the minor species of $Fc_{\varepsilon}R$ detected by the same monoclonal antibodies after surface iodination and immunoprecipitation of the $Fc_{\varepsilon}R$ utilizing the same antibodies, suggesting that the 46 and 25 kd moieties are antigenically identical, and the latter being the water-soluble part of $Fc_{\varepsilon}R$.

Although the purification of the IgE binding activity from RPMI-8866 cell culture supernatants entailed many steps, it was important to check for the immunological activity of the putative $Fc_{\varepsilon}R$ at various stages of purification. In fact equal amounts of HPLC purified $Fc_{\varepsilon}R$ were reapplied to specific 3-5-immunoaffinity and non-specific NMIg-Sepharose gels and the activity was monitored in the effluent and eluate of these gels. The results shown in Table 2 indicate that the purified material obviously binds to the specific column and almost all the activity is recovered in the eluate, compared to the non-specific gel where all the disposable activity is found in the effluent and a minor portion in the eluate. It can also be seen that a good portion of the activity is loosely associated to the non-specific gel and lost during washing.

## b) Partial sequencing of water-soluble part of Fc$_\varepsilon$R

Fc$_\varepsilon$R prepared after sequential purification of concentrated cell culture supernatant utilizing immunoaffinity gels and C-4 HPLC corresponding to a single band on SDS-PAGE and active in the ELISA assay was subjected to amino acid sequencing. The first attempts indicated that the N-terminal was blocked and therefore two different proteolytic preparations were made utilizing trypsin and lysylendopeptidase. A total of 11 and 12 fragments were obtained with trypsin and lysylendopeptidase treatment respectively. The HPLC profile of the lysylendopeptidase fragmentation shown on figure 5 indicate 12 major peaks corresponding to defined fragments of Fc$_\varepsilon$R. The following selected fragments were obtained:

Met-Glu-Leu-Gln-Val-Ser-Ser-Gly-Phe-Val-(N-terminal),

Gly-Glu-Phe-Ile-Trp-Val-Asp-Gly-Ser-His-Val-Asp-Tyr-Ser-Asn-Trp-Ala-Pro-Gly-Glu-Pro-Thr-,

Lys-His-Ala-Ser-His-Thr-Gly-Ser-Trp-Ile-Gly-Leu-Arg-Asn-Leu-Asp-Leu-Lys- and

Lys-Trp-Ile-Asn-Phe-Gln-.

## c) Preparation of the hybridization probes

Probe I (cDNA specific to Fc$_\varepsilon$R positive L cells):

Thymidine kinase (TK) deficient L cells, Ltk⁻ cells, were co-transfected with high molecular weight DNA from RPMI-8866 cells and the Herpes-simplex virus-derived TK gene. After HAT selection, TK positive transformants were stained with biotinated anti-Fc$_\varepsilon$R antibody (8-30) and FITC avidin and sorted by a cell sorter. Fc$_\varepsilon$R positive L cells were enriched by several cycles of sorting. Two L cell transformed lines, L-V-8-30 and L-VI-8-30, which express $Fc_\xi R$ detected by anti-$Fc_\xi R$(8-30), were established from two independent transfection experiments. Fig. 6 shows FACS analysis of L-V-8-30 cells which were stained with anti-$Fc_\xi R$ as well as human IgE.

The total RNA was prepared from L-V-8-30 cells by guanidine isothiocyanate/cesium chloride method (see Biochemistry 18, 5294 (1979)). The DNA complementary to mRNA from L-V-8-30 cells was synthesized using the enzyme reverse transcriptase on an oligo (dt) primer. The cDNA was labeled by incorporation of $\alpha$-$^{32}$P-deoxy CTP in this reaction. The $^{32}$P labelled cDNA was mixed and hybridized with 10 fold excess poly(A)$^+$ RNA derived from Ltk$^-$ cells. The above mixture was applied on hydroxyapatite column and the unbound single strand cDNA was rehybridized with poly(A)$^+$ RNA from Ltk$^-$ cells. The single strand cDNA which is specific to $Fc_\xi R$ positive L cell transformant was used as probe to detect the gene for $Fc_\xi R$ in $\lambda$gt 10 library (Fig. 7).

Probe II: A mixture of oligonucleotides of formula

$$3'-TT^T_C \ ACC \ TA^T_AG \ TT^A_G \ AA^A_G \ GT \ -5'$$

was synthesized by means of an ADI-synthesizer at the 0,2 µMol level according to known methods. The obtained oligonucleotide encoding partially for the amino acid sequence of formula

Lys-Trp-Ile-Asn-Phe-Gln

was used as labeled probe to detect the gene for $Fc_\xi$-receptor in an expression vehicle.

- 9 -                                    0258489

d) Isolation and identification of expression vehicles containing the gene coding for human low affinity $Fc_\varepsilon R$

The exponentially growing RPMI-8866 cells are disrupted in guanidium isothiocyanate solution. The mRNA is isolated by centrifugation on cesium chloride gradient, phenol extraction and ethanol precipitation. Then, the poly(A)$^+$ RNA is isolated by oligo (dt) cellulose chromatography.

The construction of double-stranded cDNA is carried out according to Gubler and Hoffman (Gene 25, 263 (1983)). The poly(A)$^+$ RNA is used as a matrix for the preparation of single-strand cDNA by using reverse transcriptase and an oligo (dt) primer. After treatment with RNase H, the second strand of DNA is synthesized by means of DNA polymerase I. The synthesis of first and second strand of DNA was carried out in a solution containing a mixture of deoxynucleotide triphosphate of adenosine, thymidine, guanosine and cytidine. The double stranded cDNA mixture obtained was then modified by means of the enzyme T4 DNA polymerase to remove any small remaining 3' overhangs from the first strand cDNA. The EcoRI linkers were added and ligated to the double-stranded cDNA by using the enzyme T4 ligase. The cDNA longer than 1000 bp are fractionated and excess linkers were removed by a Bio-Gel A-50 m column chromatography. The size fractionated cDNA were ligated to EcoRI digested $\lambda$gt 10 phage vector DNA. The $\lambda$gt 10 vectors containing the cDNA were packaged in vitro and infected to Escherichia coli strain 0600 hfl.

Among the plaques thus obtained, those which contain sequences specific to $Fc_\varepsilon R$ were identified by colony hybridization, whereby two different probes were used:

1. $Fc_\varepsilon R^+$ transformant-specific cDNA.

2. Radioactively labeled synthetic oligonucleotides of formula

$$3'\text{-}TT\genfrac{}{}{0pt}{}{T}{C}\ ACC\ TA\genfrac{}{}{0pt}{}{T}{A}G\ TT\genfrac{}{}{0pt}{}{A}{G}\ AA\genfrac{}{}{0pt}{}{A}{G}\ GT\ \text{-}5'$$

23 from approximately 300 000 clones hybridizing with both probes were identified and all cDNA inserts hybridized to each other. Among the cDNA's in those clones, the largest cDNA insert (approximately 1600 kb) was elected.

The EcoRI insert from the $\lambda$gt 10 recombinant DNA clone was labeled by nick translation using $\alpha\text{-}^{32}P\text{-}dCTP$ and analysed by Northern hybridization with mRNA from various cells including RPMI-8866, Daudi, CEM, $Fc_\varepsilon R^+$ L cells and $Ltk^-$ cell. The insert hybridized only with mRNA from $Fc_\varepsilon R$ positive cells such as RPMi-8866 and $Fc_\varepsilon R^+$ L cells. This insert was cloned in an EcoRI site of pGEM4 vector (Promega Biotec), named as $pFc_\varepsilon R\text{-}1$ and propagated.

## e) Expression of the $Fc_\varepsilon R$ cDNA:

The EcoRI insert containing $Fc_\varepsilon R$ cDNA, which was isolated from the above described $\lambda$gt10 clone, was ligated to EcoRI digested pGEM$^{TM}$—4 plasmid vector (see Fig. 9), named as LE392 and deposited in E.coli on August 01, 1986 under number FERM BP-1116 (Fermentation Research Institute, Agency of Industrial Science and Technology, Japan) according to the convention of Budapest. Since this vector contains both SP6 and T7 promotors in opposite orientation to each other, $Fc_\varepsilon R$ cDNA can be readily transcribed into mRNA either by SP6 or T7 RNA-polmerase. Therefore, pGEM4-DNA containing $Fc_\varepsilon R$ cDNA was digested with BamHI and the obtained plasmid DNA was used as a template to synthesize the mRNA by SP6 RNA polymerase, and the resulting RNA (5 µg) was injected into Xenopus oocytes. After 2 days of incubation, the oocytes were lysed and the presence of $Fc_\varepsilon R$ was determined by an enzyme linked immunosorbent assay (ELISA) utilizing two anti-$Fc_\varepsilon R$ antibodies,

3-5 and 8-30, which recognize different epitopes on $Fc_{\varepsilon}R$. As shown in Fig. 9, the lysate of ten oocytes injected with the RNA transcript of $pFc_{\varepsilon}R$-1 showed $Fc_{\varepsilon}R$ levels comparable to that derived from $1 \times 10^5$ RPMI-8866 cells. On the other hand, the lysate of mock-injected oocytes did not show any activity. This result indicates that the product of $pFc_{\varepsilon}R$-1 cDNA shares the two different antigenic determinants with $Fc_{\varepsilon}R$ recognized by the monoclonal antibodies.

A further expression vector, for example pDE2 (see Japanese Patent Publication 1986/88879 from May 7, 1986), which contains two SV40 early promotors in opposite orientation to each other to ensure the cDNA expression in either orientation (Fig. 8) was employed to confirm that $pFc_{\varepsilon}R$-1 includes the entire coding sequence of $Fc_{\varepsilon}R$. The segment of DNA between the two SV40 early promoters was removed by EcoRI digestion and replaced with the insert cDNA of $pFc_{\varepsilon}R$-1 ($pDE2$-$Fc_{\varepsilon}R$-1). Cos7 cells were transfected with 2 µg/ml of pDE2 containing $Fc_{\varepsilon}R$ cDNA by the DEAE-dextran method. After 2 days culture, cells were doubly stained with anti-$Fc_{\varepsilon}R$ and human IgE and analysed on a dual laser FACS. As shown in Fig. 8', approximately 30 % of the cells transfected with $pDE2$-$Fc_{\varepsilon}R$-1 were labeled by both anti-$Fc_{\varepsilon}R$ and human IgE. Furthermore, the staining with anti-$Fc_{\varepsilon}R$ and human IgE was well correlated, demonstrating that both anti-$Fc_{\varepsilon}R$ and IgE bound to the same molecule(s) that is newly expressed on the surface of transfected cells. Indeed, cells transfected with the control pDE2 vector containing human IFN-ß cDNA were not stained with either anti-$Fc_{\varepsilon}R$ or human IgE. These results confirmed that the isolated cDNA actually encodes the $Fc_{\varepsilon}R$ molecule.

f) Determination of the complete nucleotide sequence of the $Fc_{\varepsilon}R$ cDNA and the deduced protein sequence

The complete nucleotide sequence of the EcoRI insert from

pFc$_t$R-1 was determined using the dideoxy termination method (see Sanger et al. in Proc. Natl. Acad. Sci. USA 74, 5463-5467 (1977)) and the chemical cleavage method (see Maxam and Gilbert in Proc. Natl. Acad. Sci. USA 74, 560-564 (1977)). The complete nucleotide sequence and the deduced amino acid sequence are shown in Table 3, whereby the coding sequence shows the following formula:

```
                    5                   10                  15
Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG
TAC CTC CTT CCA GTT ATA AGT CTC TAG CTC CTC GAA GGG TCC TCC

                    20                  25                  30
Arg Cys Cys Arg Arg Gly Thr Gln Ile Val Leu Leu Gly Leu Val
CGG TGT TGC AGG CGT GGG ACT CAG ATC GTG CTG CTG GGG CTG GTG
GCC ACA ACG TCC GCA CCC TGA GTC TAG CAC GAC GAC CCC GAC CAC

                    35                  40                  45
Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG
TGG CGG CGA GAC ACC CGA CCC GAC GAC TGA GAC GAA GAG GAC ACC

                    50                  55                  60
His Trp Asp Thr Thr Gln Ser Leu Lys Gln Leu Glu Glu Arg Ala
CAC TGG GAC ACC ACA CAG AGT CTA AAA CAG CTG GAA GAG AGG GCT
GTG ACC CTG TGG TGT GTC TCA GAT TTT GTC GAC CTT CTC TCC CGA

                    65                  70                  75
Ala Arg Asn Val Ser Gln Val Ser Lys Asn Leu Glu Ser His His
GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

                    80                  85                  90
Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

                    95                  100                 105
Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

                    110                 115                 120
Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC
```

```
                      125                      130                      135
 Ser Ser Phe Lys Ser Gln Glu Leu Asn Glu Arg Asn Glu Ala Ser
 AGC AGC TTC AAG TCC CAG GAA TTG AAC GAG AGG AAC GAA GCT TCA
 TCG TCG AAG TTC AGG GTC CTT AAC TTG CTC TCC TTG CTT CGA AGT

                      140                      145                      150
 Asp Leu Leu Glu Arg Leu Arg Glu Glu Val Thr Lys Leu Arg Met
 GAT TTG CTG GAA AGA CTC CGG GAG GAG GTG ACA AAG CTA AGG ATG
 CTA AAC GAC CTT TCT GAG GCC CTC CTC CAC TGT TTC GAT TCC TAC

                      155                      160                      165
 Glu Leu Gln Val Ser Ser Gly Phe Val Cys Asn Thr Cys Pro Glu
 GAG TTG CAG GTG TCC AGC GGC TTT GTG TGC AAC ACG TGC CCT GAA
 CTC AAC GTC CAC AGG TCG CCG AAA CAC ACG TTG TGC ACG GGA CTT

                      170                      175                      180
 Lys Trp Ile Asn Phe Gln Arg Lys Cys Tyr Tyr Phe Gly Lys Gly
 AAG TGG ATC AAT TTC CAA CGG AAG TGC TAC TAC TTC GGC AAG GGC
 TTC ACC TAG TTA AAG GTT GCC TTC ACG ATG ATG AAG CCG TTC CCG

                      185                      190                      195
 Thr Lys Gln Trp Val His Ala Arg Tyr Ala Cys Asp Asp Met Glu
 ACC AAG CAG TGG GTC CAC GCC CGG TAT GCC TGT GAC GAC ATG GAA
 TGG TTC GTC ACC CAG GTG CGG GCC ATA CGG ACA CTG CTG TAC CTT

                      200                      205                      210
 Gly Gln Leu Val Ser Ile His Ser Pro Glu Glu Gln Asp Phe Leu
 GGG CAG CTG GTC AGC ATC CAC AGC CCG GAG GAG CAG GAC TTC CTG
 CCC GTC GAC CAG TCG TAG GTG TCG GGC CTC CTC GTC CTG AAG GAC

                      215                      220                      225
 Thr Lys His Ala Ser His Thr Gly Ser Trp Ile Gly Leu Arg Asn
 ACC AAG CAT GCC AGC CAC ACC GGC TCC TGG ATT GGC CTT CGG AAC
 TGG TTC GTA CGG TCG GTG TGG CCG AGG ACC TAA CCG GAA GCC TTG

                      230                      235                      240
 Leu Asp Leu Lys Gly Glu Phe Ile Trp Val Asp Gly Ser His Val
 TTG GAC CTG AAG GGA GAG TTT ATC TGG GTG GAT GGG AGC CAT GTG
 AAC CTG GAC TTC CCT CTC AAA TAG ACC CAC CTA CCC TCG GTA CAC

                      245                      250                      255
 Asp Tyr Ser Asn Trp Ala Pro Gly Glu Pro Thr Ser Arg Ser Gln
 GAC TAC AGC AAC TGG GCT CCA GGG GAG CCC ACC AGC CGG AGC CAG
 CTG ATG TCG TTG ACC CGA GGT CCC CTC GGG TGG TCG GCC TCG GTC

                      260                      265                      270
 Gly Glu Asp Cys Val Met Met Arg Gly Ser Gly Arg Trp Asn Asp
 GGC GAG GAC TGC GTG ATG ATG CGG GGC TCC GGT CGC TGG AAC GAC
 CCG CTC CTG ACG CAC TAC TAC GCC CCG AGG CCA GCG ACC TTG CTG

                      275                      280                      285
 Ala Phe Cys Asp Arg Lys Leu Gly Ala Trp Val Cys Asp Arg Leu
 GCC TTC TGC GAC CGT AAG CTG GGC GCC TGG GTG TGC GAC CGG CTG
 CGG AAG ACG CTG GCA TTC GAC CCG CGG ACC CAC ACG CTG GCC GAC
```

```
                    290                      295                      300
Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC


                    305                      310                      315
Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG


Ser Ala Pro Leu His Ser
TCT GCC CCT CTC CAC TCT TGA
AGA CGG GGA GAG GTG AGA ACT
```

The single large open reading frame begins at position 186 and extends 963 nucleotides, encoding 321 amino acids. The isolated partial amino acid sequences of three peptide fragments and the isolated N-terminal Met-Glu-Leu-Gln-Val-Ser-Ser-Gly-Phe-Val- of purified Fc R confirm that the longest frame is the coding sequence of Fc R protein. The hydrophilic N-terminal sequence consisting of 21 amino acids is followed by a hydrophobic region which consists of 26 uncharged amino acids (22-47). The signal sequence, typicall located in the N-terminus of most membrane-bound or secretory proteins, was not found. Hence, the hydrophobic stretch of 26 amino acids is likely to be a membrane-embedded region, since the subsequent residues are mostly hydrophilic and no other part of the sequence appears likely to cross the membrane. The N-terminal hydrophilic stretch is terminated by a cluster of very basic amino acid residues (Arg-Arg-Arg-Cys-Cys-Arg-Arg). This cluster of basic amino acids usually found on the cytoplasmic side of membrane proteins is known to be a stop-transfer sequence which has an important role in integration into the lipid bilayer (see Blobel in Proc. Natl. Acad. Sci. USA 77, 1496-1500 (1980) and Schneider et al. in Nature 311, 675-678 (1984)). There is one putative N-linked carbohydrate addition site at position 63 which should be located in the extracellular region for membrane proteins. All of these results demonstrate that Fc R is oriented with the N-terminus on the cytoplasmic side and the C-terminus outside the cell.

Relatively large amounts of soluble 25 kd Fc R were found in the culture supernatant of RPMI-8866 cells. The N-terminal amino acid residue (Met) of the soluble $Fc_\epsilon R$ was found at position 150, and the preceeding residue, arginine is a common target for trypsin-like proteases. The C-terminal region (150-321) contains two clusters of cysteins (160, 163, 174 and 191 and 259, 273, 282 and 288) which probably from disulfide bonds and results in a tightly folded structure, which will be resistant to proteolytic enzymes. The C-terminal region (150-321) corresponds therefore, to the soluble $Fc_\epsilon R$ which is a product of proteolytic cleavage of the membrane-bound $Fc_\epsilon R$.

g) Expression of the $Fc_\epsilon R$ mRNA:

The poly(A)$^+$ RNA was prepared from various types of cells and analyzed for expression of $Fc_\epsilon R$ mRNA by Northern blotting, whereby a major band of 1,700 b was detected in B lymphoblastoid cell lines (RPMI-8866, RPMI-1788), fetal liver-derived Epstein Barr virus transformed pre B cell line (FL #8-2) and two $Fc_\epsilon R^+$ L cell transformants, but not in $Fc_\epsilon R^-$ cells including two Burkitt's lymphoma lines (Daudi and Jijoye), a T cell line (CEM) and a L cell transformant which expresses another B Cell antigen (CD20). Furthermore, $Fc_\epsilon R$ mRNA was not detected in normal T cells, whereas normal B cells expressed a comparable level of $Fc_\epsilon R$ mRNA as B lymphoblastoid lines.

h) Preparation of an expression vector containing the DNA-sequences coding for a water soluble fragment of $Fc_\epsilon$-receptor

From the obtained gene for $Fc_\epsilon$-receptor (see table 3) were removed by means of a suitable endonuclease the codons coding for the water-insoluble part of $Fc_\epsilon$-receptor, conveniently the codons for the amino acids from about 50 to 150, preferably from about 150. When introducing the obtained shorte-

ned Fc$_\xi$-receptor genes into organisms under condition which lead to high yield thereof, there were obtained the desired water-soluble polypeptides without the above mentioned amino acids. Therefore, the water soluble part of Fc$_\xi$-receptor contains at least the follwing sequence:

```
                                                            Met
                                                            ATG
                                                            TAC

Glu Leu Gln Val Ser Ser Gly Phe Val Cys Asn Thr Cys Pro Glu
GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

Thr Lys Gln Trp Val His Ala Arg Tyr Ala Cys Asp Asp Met Glu
ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

Gly Gln Leu Val Ser Ile His Ser Pro Glu Glu Gln Asp Phe Leu
GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

Thr Lys His Ala Ser His Thr Gly Ser Trp Ile Gly Leu Arg Asn
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

Leu Asp Leu Lys Gly Glu Phe Ile Trp Val Asp Gly Ser His Val
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

Gly Glu Asp Cys Val Met Met Arg Gly Ser Gly Arg Trp Asn Asp
GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG
```

```
Ser Ala Pro Leu His Ser
TCT GCC CCT CTC CAC TCT TGA
AGA CGG GGA GAG GTG AGA ACT
```

However, the before mentioned polypeptide is the most important one of the water-soluble species of human low affinity Fc$_\xi$R. The corresponding genes can be introduced into organisms under conditions which lead to high yields thereof, as mentioned hereinbefore. Useful hosts and vectors are well known to those of skill in the art, and reference is made, for example, to European Patent Publication 0 093 619 published November 9, 1983.

In general, prokaryotes are preferred for expression. For example, E. coli K12 strain 294 (ATCC No. 31446) is particularly useful. Other microbial strains which may be used include E. coli X1776 (ATCC No. 31537). The aforementioned strains, as well as E. coli W3110 (F⁻, lambda⁻, prototrophic, ATCC No. 27325), bacilli such as Bacillus subtilus, and other enterobacteriaceae such as Salmonella typhimurium or Serratia marcenses, and various Pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an Escherichia coli species (Bolivar, et al., Gene 2: 95 (1977)). pBR322 contains genes for ampicillin and tetracyline resistance and thus provides easy means for identifying transformed cells. The pBR322 plasmid, or other microbial plasmids must also contain, or be modified to contain, promotors which can be used by the microbial organism for expression. Those promotors most commonly used in recombinant DNA construction include the beta-lactamase (penicilli-nase) and lactose promotor systems (Chang et al., Nature 275, 615 (1978); Itakura et al., Science 198, 1056 (1977); Goeddel et al., Nature 281, 544 (1979)) and tryptophan (trp) promotor system (Goeddel et al., Nucleic Acids Res. 8, 4057 (1980); EP-A-0 036 776). While these are the most commonly used, other microbial promotors have been discovered and utilized.

For example, the genetic sequence for $Fc_\varepsilon R$ can be placed under the control of the leftward promotor of bacteriophage Lambda ($P_L$). This promotor is one of the strongest known promotors which can be controlled. Control is exerted by the lambda repressor, and adjacent restriction sites are known. A temperature sensitive allele of this repressor gene can be placed on the vector that contains the complete $Fc_\varepsilon R$ sequence. When the temperature is raised to 42°C, the repressor is inactivated, and the promotor will be expressed at its maximum level. The amount of mRNA produced under these conditions should be sufficient to result in a cell which contains about 10 % of its newly synthesized RNA originated from the $P_L$ promotor. In this scheme, it is possible to establish a bank of clones in which a functional $Fc_\varepsilon R$ sequence is placed adjacent to a ribosome binding sequence, and at varying distances from the lambda $P_L$ promotor. These clones can then be screened and the one giving the highest yield selected.

The expression and translation of the $Fc_\varepsilon R$ sequence can also be placed under control of other regulons which may be "homologous" to the organism in its untransformed state. For example, lactose dependent E. coli chromosomal DNA comprises a lactose or lac operon which mediates lactose digestion by expressing the enzyme beta-galactosidase. The lac control elements may be obtained from bacteriophage lambda plaC5, which is infective for E. coli. The phage's lac operon can be derived by transduction from the same bacterial species. Regulons suitable for use in the process of the invention can be derived from plasmid DNA native to the organism. The lac promoter-operator system can be induced by IPTG.

Other promoter-operator systems or portions thereof can be employed as well. For example, the arabinose operator, Colicine $E_1$ operator, galactose operator, alkaline phosphatase operator, trp operator, xylose A operator, tac promotor, and the like can be used.

The genes can be advantageously expressed in expression plasmid pER103 (E. Rastl-Dworkin et al., Gene 21, 237-248 (1983); see also European Patent Application No. 83 112 812.9, dated December 20, 1983 Deposit DSM 2773). This vector contains all regulatory elements which lead to a high expression rate of the cloned gene.

In addition to prokaryotes, eukaryotic microbes, such as yeast cultures may also be used. Saccharomyces cerevisiae is the most commonly used among eukaryotic microorganisms, although a number of other species are commonly available. For expression in Saccharomyces, the plasmid YRp7, for example (Stinchcomb, et al., Nature 282, 39, (1979); Kingsman et al., Gene 7, 141 (1979); Tschumper, et al., Gene 10, 157 (1980)) and plasmid YEp13 (Bwach et al., Gene 8, 121-133 (1979)) are commonly used. The plasmid YRp7 contains the TRP1 gene which provides a selection marker for a mutant strain of yeast lacking the ability to grow in tryptophan, for example ATCC No. 44076. The presence of the TRP1 lesion as a charactristic of the yeast host cell genome then provides an effective environment for detecting transformation by growth in the absence of tryptophan. Similarly, the plasmid YEp13 contains the yeast LEU2 gene which can be used for complementation of a LEU2 minus mutant strain.

Suitable promoting sequence in yeast vectors include the
5'-flanking region of the genes for ADH I (Ammerer, G., Methods of Enzymology 101, 192-201 (1983)), 3-phosphoglycerate
kinase (Hitzemann, et al., J. Biol. Chem. 255, 2073 (1980))
or other glycolytic enzymes (Kawasaki and Fraenkel, BBRC
108, 1107-1112 (1982)), such as enolase, glyceraldehyde-3-
phosphate dehydrogenase, hexokinase, pyruvate decarboxylase,
phosphofructokinase, glucose-6-phosphate isomerase, phospho-
glucose isomerase, and glucokinase. In constructing suitable
expression plasmids, the termination sequences associated
with these genes are also ligated into the expression vector
3' end of the sequence desired to be expressed, to provide
polyadenylation of the mRNA and termination.

Other promotors, which have the additional advantage of
transcription controlled by growth conditions are the promotor regions of the genes for alcohol dehydrogenase-2, iso-
cytochrome C, acid phosphatase, degradative enzymes associated with nitrogen metabolism, the aforementioned glyceraldehyde-3-phosphate dehydrogenase, and enzymes responsible for
maltose and galactose utilization. Promotors which are regulated by the yeast mating type locus, such as the promotors
of the genes BARI, MR-alpha-1, STE2, STE3, STE5 can be used
for temperature regulated system by using temperature dependent siv mutations (Rhine, Ph. D. Thesis, University of Oregon, Eugene, Oregon (1979), Herskowitz and Oshima in The
Molecular Biology of the Yeast Sacharomyces, Part I, 181-209
(1981), Cold Spring Harbor Laboratory). These mutations directly influence the expressions of the silent mating type
cassettes of yeast, and therefore indirectly the mating type
dependent promotors.

Generally, however, any plasmid vector containing a yeast
compatible promotor, origin of replication and termination
sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years (Tissue Culture, Academic Press, Kruse and Patterson, Editors (1973)). Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promotor located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promotors are derived from polyoma, Adenovirus 2, and most frequently Simian Virus 40 (SV40). The early and late promotors of SV40 are particularly useful because both are obtained easily from the virus as a fragment which also contains the SV40 viral origin of replication (Fiers et al., Nature 273, 113 (1978)). Smaller or larger SV40 fragments may also be used, provided there is included the approximately 250 bp sequence extending from the Hind III site toward the Bgl I site location in the viral origin of replication. Further, it is also possible, and often desirable, to utilize promotor or control sequences normally associated with the desired gene sequence, provided such control sequences are compatible with the host cell systems.

An origin of replication may be provided either by construction of the vector to include an exogenous origin, such as may be derived from SV40 or other viral (e.g., Polyoma,

0258489

Adeno, VSV, BPV, etc.) source, or may be provided by the host cell chromosomal replication mechanism. If the vector is integrated into the host cell chromosome, the latter is often sufficient.

i) The prepared human lower affinity $Fc_\xi$-receptor prepared according to the invention, preferably the water-soluble part thereof, is suitable for the treatment of local and allergic reactions induced by expression of IgE and may be incorporated in the suitable pharmaceutical compositions such as solutions or sprays.

The following examples, which are not exhaustive, will illustriate the invention in greater detail:

General Materials and Methods:

The monoclonal anti-$Fc_\xi$R antibodies 3-5 ($\gamma_1$) and 8-30 ($\mu$) were produced by hybridization of P3U1 myeloma with spleen cells from Balb/c mice immunized with RPMI-8866 cells (see European Patent Application No. 86 110 420.6 of the same Applicant, filed on July 29, 1986). The 8-30 antibody recognizes the epitope close to IgE binding site of $Fc_\xi$R and could block binding of IgE to 8866 lymphoblastoid cells. The 3-5 antibody, recognizes a different epitope on $Fc_\xi$R and can not block effectively IgE binding to its receptors. These antibodies precipitate 46 kd and 25 kd polypeptides under reducing and non reducing conditions. The monoclonal antibodies were purified from ascitis by 50 % saturated ammonium sulfate precipitation followed by gel filtration using Sepharose 6B (Pharmacia Fine Chemical, Uppsala, Sweden) for IgM class or ion exchange chromatography using QAE-Sephadex (Pharmacia Fine Chemical) for IgG1. The polyclonal mouse IgG was isolated in the same fashion. The anti-mouse IgM-alkaline phosphatase conjugate was purchased from Tago (Burlingame, CA).

Reverse phase HPLC was carried out by using a Waters HPLC system with Hi-Pore, RP-304 (250 x 4.6 mm) (Bio-Rad) column. The immunoaffinity purified $Fc_\xi R$ was applied to the reverse phase column equilibrated with water containing 0,1 % trifluoracetic acid and eluted with a linear gradient of acetonitril containing 0,1 % trifluoroacetic acid (TFA). A flow rate of 0.5 ml/min and a linear gradient (from 0 % to 65 % for 60 min) of acetonitril was employed. The eluted material was frozen and lyophilized prior to testing for activity in ELISA.

## $NaDodSO_4$/PAGE

Crude, immunoaffinity purified and HPLC purified fractions of $Fc_\xi R$ were analyzed by electrophoresis on a 1 % $NaDodSO_4$/ 10 % polyacrylamide gel (Fig. 4) and proteins were determined by silver staining using Daiichikagaku silver stain (Daiichi-Kagaku, Tokyo). To measure Fc R activity, after electrophoresis, the gel was cut into 4 mm slices, minced eluted with lysis buffer overnight at room temperature with shaking, the eluted material was collected after centrifugation and tested for activity in ELISA.

## Example 1

### a) Isolation of crude $Fc_\xi R$ from culture supernatant

RPMI-8866 cells were cultured in RPMI1640 medium supplemented with 10 % fetal calf serum and antibiotics (100 units/ml of penicillin and 100 μg/ml of Streptomycin) at a density of $1 \times 10^6$ cells/ml and a cell viability of 95-99 % in Spinner bottles. The cells were harvested after 15 min centrifugation at 5,000 rpm washed 3 times with Hank's BSS and cultured at the same density in serum-free medium for 48 hours in Spinner bottles. The culture supernatant was collected and supplemen-

ted with phenylmethyl sulfonylfluoride (PMSF) (1 mM), 0,02 %
sodium azide ($NaN_3$), and 200 units/ml of aprotenin. The
culture supernatants were stored at 4°C during concentration.

Concentration of RMPI-8866 culture sups was carried out by
an Amicon filter system using a DIAFLQ, YM10-filter. The
200-300 times concentrated material was then centrifuged at
85,000 x G for 40 min at 4°C in order to remove insoluble
material, whereby crude $Fc_\varepsilon R$ preparation was obtained.

b) Isolation of $Fc_\varepsilon R$ from cell lysates

RMPI-8866 cells (2 x $10^9$ cells) were washed 4 times with
PBS and lysed in 10 ml of lysis buffer (PBS containing 0,5 %
Nonindet P-40 (NP-40). 1mM PMSF for 45 min on ice with periodic vortexing. An additional 10 ml of lysis buffer was
added and the lysis continued for an additional 30 min on
ice. The lysate was centrifuged at 37.000 rpm for 45 min at
4°C. The lipid layer was carefully removed and the supernatant collected and stored at -70°C.

Example 2

Immunoaffinity Purification

Culture supernatant concentrate (see Example 1a) equivalent
to 5-10 liters of culture were sequentially adsorbed on 2 ml
of BSA-Sepharose, human transferrin-Sepharose and normal
mouse Ig (NMIg)-Sepharose gels for one hour each at 4°C with
rotation. The effluent collected from the last immunoaffinity gel was then applied on 2 ml of anti-$Fc_\varepsilon R$(3-5) coupled to
Sepharose. Immunoadsorption was allowed to proceed between
4-16 hours at 4°C with rotation. The gel was poured into a
column, the effluent collected and the gel washed sequentially with 150 ml of buffer A (Tris-HCL, 10 mM, pH 7.5, NaCl, 0.5 M, NP-40, 0.5 %), 150 ml of buffer B (Tris-HCl, 10 mM, pH 7.5, NP.40 0,1 %), 150 ml of buffer C (Tris-HCl, 10 mM, pH 7.5) and eluted with 25 ml of 2.5 % acetic acid (v/v) and immediately neutralized in Tris-HCl, 2 M, pH 8.0. The material was stored at -80°C if not used immediately for further purification utilizing HPLC. - Then, the eluate was fractionated by a reverse phase HPLC on a C4 column utilizing a linear gradient of 0-65% acetonitril containing 0,1% trifluoroacetic acid.

Example 3

Enzyme Linked Immunosorbent Assay (ELISA)

The $Fc_\epsilon R$ activity was measured by its ability to bind to the monoclonal antibodies 3-5 and 8-30 and monitored utilizing a double antibody enzyme-linked immunosorbent assay (ELISA). 96 well microtiter plates were initially coated with the monoclonal antibody 3-5 100 µl/well (10 µg/ml) in coating buffer ($NaHCO_3$ 0.1 M, pH 9.6), and incubated overnight at 4°C. The plates were then washed 4 times with rinse buffer, i.e. Dulbecco's, phosphate buffer pH containing 0,05 % Tween 20, followed by the addition of 100 µl test sample diluted with diluent buffer (Tris-HCl 0.05 M, pH 8.1, $MgCl_2$ 1mM, NaCl 0,15 M, Tween 20 0.05 % (v/v), $NaN_3$ 0.02 %, BSA 1 %). The microtiter plates were incubated for 2 hours at room temperature, and washed 4 times with rinse buffer, followed by the addition of 100 µl of a pretitrated and diluted goat-anti-mouse IgM-alkaline phosphatase conjugates. The plates were incubated for two hours at room temperature and washed 4 times with rinse buffer. In the final step 200 µl of substrate, p-phenyl phosphate disodium (1 mg/ml) in substrate buffer ($NaHCO_3$ 0.05 M, pH 9.8, $MgCl_2$ x 6 $H_2O$, 10 mM) was added and the colorimetric reaction measured every 30 min for two hours at 405 and 620 nm.

## Example 4


## Hydrolysis of Fcε-receptor by means of lysylendopeptidase and Separation of Peptides

The purified FcεR was digested with Achromobacter lyticus lysylendopeptidase in 50 mM Tris-HCl buffer, pH 9.5 for 6 hours at 35°C at an enzyme-to-substrate ratio of 1:500 (W/W). The lyophilized peptide fragments were purified by HPLC.


## Separation of Peptides by Reverse Phase HPLC

Separation of peptides was performed by HPLC using a C4 column on a Hitachi 655 liquid chromatograph equipped with a 655-60 gradient processor and a Rheodyne Sample injector with a 100 µl sample loop. The elution of peptides was carried out with a linear gradient of 2-propanol: acetonitril = 7:3 (v/v) from 0-35 % for 1 hour in 0.1 % trifluoracetic acid and a flow rate of 1.0 ml/min.. The fractionated peptides were manually collected by monitoring the absorbance at 215 nm.


## Amino Acid Analysis and Sequence Determination

The amino acid analyses were carried out with a Hitachi 835-5 amino acid analyzer after hydrolysis of the peptide fragments in 5.7 HCl at 110°C in evacuated, sealed tubes for 22-24 hours. Automated Edman degradation was performed with a 470 A protein sequencer (Applied Biosystems, Inc. CA) using a standard program for sequencing. The phenylthiohydantoin (PTH)-amino acids were determined by reverse phase HPLC with isooratic elution (see Tsunasawa et al. in J. Biochem. 97, 701-704 (1985)).

The following amino acid sequences were detected:

Met-Glu-Leu-Gln-Val-Ser-Ser-Gly-Phe-Val-,

Gly-Glu-Phe-Ile-Trp-Val-Asp-Gly-Ser-His-Val-Asp-Tyr-Ser-Asn-Trp-Ala-Pro-Gly-Glu-Pro-Thr-,

Lys-His-Ala-Ser-His-Thr-Gly-Ser-Trp-Ile-Gly-Leu-Arg-Asn-Leu-Asp-Leu-Lys- and

Lys-Trp-Ile-Asn-Phe-Gln-.

Example 5

Preparation of the oligonucleotide of formula

$$3'-TT^T_C \; ACC \; TA^T_AG \; TT^A_G \; AA^A_G \; GT \; -5'$$

The oligonucleotide was synthesized using an ADI-synthesizer at the 0,2 µMol level.

The resulting oligonucleotide was demethylated with thiophenol/triethylamin/dioxan (1:2:2) at room temperature within 90 minutes and washed with methanol (10 x 1 ml of methanol).

After removing the demethylated oligonucleotide from controlled pore glass (CPG) and splitting off the protective group with concentrated ammonia within 1 hour at room temperature and 16 hours at 55°C, the oligonucleotide was dried by means of Speedvac ®.

The further purification was carried out over 20 % of acrylamide/8 Mol of urea gel with TBE-buffer (10,9 g of tris (hydroxymethyl)aminomethane; 5,5 g of boric acid and 9,3 g of Ethylenedinitrilo-tetraacetic acid disodium salt in 1 1).

The subsequent gel-electrophorese (40 cm x 25 cm x 0,1 cm) was carried out at 50 watt. The 17-mer band was cut out, eluted with water and desalted on a 0,9 x 13 cm Sephadex G 25 medium column in water.

The fractions containing the 17-mer were pooled and dried. Yield: 7,7 $OD_{260}$ ($\sim$ 285 µg)

Example 6

Establishment of $Fc_\epsilon R^+$ L cell transformants

---

One million Ltk⁻ cells were co-transfected with 150 ng Herpes simplex virus derived tk gene and 20 µg high molecular weight genomic DNA from RPMI-8866 cells (see Wigler et al. in Cell 16, 777-785 (1978)). Cells were selected in HAT medium for 10 days. HAT resistant L cells were collected, stained with biotinated anti-$Fc_\epsilon R$ (8-30) and fluorescein isothiocyanate (FIIC) conjugated avidin and sorted by FACS. Several cycles of sorting were carried out for each transfection. Two transformant lines, L-V-8-90 and L-VI-8-30 were obtained from independent transfections.

Example 7

Cloning of the $Fc_\epsilon R$ cDNA

---

a. Probe I: Total RNA was isolated from the $Fc_\epsilon R^+$ L cell transformant L-V-8-30 by the guanidium/cesium chloride method (see Chirgwin et al. in Biochemistry 18, 5294-5299 (1979)). Poly(A)$^+$ RNA was prepared by oligo dT cellulose chromatography. Radio-labeled cDNA was synthesized from poly(A)$^+$ RNA by using $^{32}$P-dCTT (see Davis et al. in Proc. Natl. Acad. Sci. USA 81, 2194-2198 (1984)). The labelled cDNA was annealed with an excess of poly(A)$^+$ RNA of untransformed Ltk$^-$ cells and applied on hydroxyapatite column. The single-stranded cDNA was collected and used as probe I.

b. Probe II: The oligonucleotides complementary to the mRNA sequence encoding for the amino acid fragment Lys-Trp-Ile-Asn-Phe-Gln, containing a mixture of 24 possible sequences, were radiolabeled with γ-$^{32}$P-ATP by T4 polynucleotide kinase. Double-stranded cNDA was synthesized from poly(A)$^+$ RNA derived from RPMI-8866 cells using Amersham cDNA synthesis system (Amersham, UK) (see Gubler and Hoffman in Gene 25, 263-269 (1983)). After treatment with EcoRI methylase and T4 DNA polymerase, the double stranded cDNA longer than 1,000 bp was cloned into the EcoRI site of λgt10 using EcoRI linkers and packaged in vitro using Gigapack (Vector cloning systems). Two sets of replica filters of phage plaques were made. One set of filters were hybridized with $^{32}$P-labeled 17-base long oligonucleotides (see Wood et al. in Proc. Natl. Acad. Sci. USA 83, 1585-1588 (1985)). Another set of filters were hybridized with $^{32}$P-labeled subtracted cDNA specific to Fc$_\varepsilon$R positive L cells overnight in 6xSSC at 68°C and washed with 0.1xSSC and 0,1 % SDS for 2 hours. The plaques which hybridized with both probes were isolated.

Example 8

Expression of Fc$_\varepsilon$R cDNA

For expression of Fc$_\varepsilon$R cDNA in pGEM4 using SP6 promotor, mRNA was synthesized with SP6 RNA polymerase using BamHI

digested pFc$_\epsilon$R-1 as a template (see Melton et al. in Nucl. Acids. Res. 12, 7035-7056 (1984)). 10 ng mRNA were injected into one oocyte and oocytes were incubated at 20°C in Barths's medium. As a negative control, murine BSF-1 mRNA was similarly prepared and injected. After 2 days incubation at 20°C, the oocytes were lysed in 50 μl lysis buffer (10 mM Tris-HCl, pH 7.5, 0.5 M NaCl, 0,5 % NP40). The oocyte lysates were tested for Fc$_\epsilon$R activity using an ELISA method consisting of a sandwich technique and using two anti Fc$_\epsilon$R antibodies, 3-5 and 8-30. As a positive control, concentrated culture supernatant from RPMI-8866 cells was employed. For expression of Fc$_\epsilon$R cDNA in Cos-7 cells, the insert of pFc$_\epsilon$R-1 was cloned into the EcoRI site of pDE2 vector (see Japanese Patent Publication 1986/88879 from May 7, 1986). Cos-7 cells (5x10$^5$) were seeded onto 60 mm plates one day prior to transfection. Transfection was carried out with 2 μg of plasmid DNA in 1 ml of 25 mM Tris-HCl (pH 7.5), 137 mM NaCl, 5 mM KCl, 0.6 mM Na$_2$HPO$_4$, 0.5 mM MgCl$_2$, 0.7 mM CaCl$_2$ and 500 μg of DEAE-dextran (Pharmacia Fine Chemical). After 1 hour incubation at 37°C, the solution was replaced with DMEM containing 10 % FCS and 150 μM chloroquine, incubated at 37°C for 3 hours and then replaced with fresh DMEM containing 10 % FCS. 48 hours later cells were stained with phycocyanin conjugated anti-Fc$_\epsilon$R antibody (3-5) and biotinated IgE, developed with FITC-avidin and analyzed by a dual laser FACS (see Kikutani et al. in J. Exp. Med. in press (1986)).

Example 9

Determination of the nucleotide sequence of the Fc$_\epsilon$R cDNA

The insert of pFc$_\epsilon$R-1 was digested with HindIII and PvuII restriction enzymes. Digested DNA was subcloned in M13 and sequenced (see Sanger et al. in Proc. Natl. Acad. Sci. USA 74, 5463-5467 (1977)) and confirmed by the procedure of Maxam and Gilbert (see Proc. Natl. Acad. Sci. USA 74, 560-564 (1977)).

Example 10

Northern blot analysis of Fc$_\varepsilon$R mRNA

Three micrograms of poly(A)$^+$ RNA from various cells were separated on 1 % agarose gel, transferred to nitrocellulose papers and hybridized with a nick translated pFc R-1 insert (see Thomas et al. in Proc. Natl. Acad. Sci. USA 77, 5201-5205 (1980)). For BSF-1 induction, one hundred million B or T cells were cultured with or without 10 µg/ml IgE and 50 µ/ml recombinant human BSF-1 (see Yokota et al. in Proc. Natl. Acad. Sci. USA in press (1986)) for 24 hours and 10 µg or total RNA was extracted and analyzed by Northern blotting as described above.

<u>Figure 1</u> shows the Fc$_\varepsilon$R activity derived from NP-40 detergent solubilized RPMI-8866 cells and serum-free culture supernatants (O———O) culture sup cell lysate (△———△).

<u>Figure 2</u> demonstrates the immunoaffinity purified soluble Fc$_\varepsilon$R. Immunoaffinity purified soluble Fc$_\varepsilon$R derived from RPMI-8866 culture supernatants was analyzed by NaDodSO$_4$/PAGE under nonreducing conditions. After electrophoresis strips of 4 mm in width were cut, minced and eluted in lysis buffer overnight at room temperature. The Fc$_\varepsilon$R activity was assessed by an ELISA method utilizing two specific monoclonal antibodies.

<u>Figure 3</u> shows the purification of water-soluble Fc$_\varepsilon$R. Serum-free culture supernatants of RPMI-8866 cells was concentrated 200 x on Amicon YM10. Sequentially preadsorbed on BSA-Sepharose, Transferrin-Sepharose, NMIg-Sepharose, followed by specific immunoaffinity chromatography on 3-5-Sepharose, Eluate applied to C-4 HPLC column and eluted with a linear gradient of acetonitril 0-65 % containing 0,1 % trifluoroacetic acid. Fractions containing Fc$_\varepsilon$R activity are indicated by hatched lines.

<u>Figure 4</u> shows the purifified water-soluble Fc$_\varepsilon$-receptor with a molecular weight of about 25 kd. The purified soluble Fc$_\varepsilon$R was examined by SDS-PAGE analysis and a photograph of the silver stained gel is shown.

<u>Figure 5</u> shows the peptide map of the water-soluble Fc$_\varepsilon$R after lysylendopeptidase digestion and was obtained after extensive preadsorption immunoaffinity chromatography and HPLC.

<u>Figure 6</u> shows the FACS analysis of L cell transformants. (Left panel A), L cell transformants, L-V-8-30 were stained with biotinated human IgG (a) or human IgE (b) and developed with FITC-avidin. (Right panel B), The same cells were stained with phycocyanin conjugated control, irrelevant murine IgG$_1$ antibodiy (c), or phycocyanin conjugated anti-Fc$_\xi$R (3-5) (d). Unstained cells showed the same pattern as those stained with control antibodies (a and c). Y and X axes represent relative cell numbers and log fluorescence intensities respectively.

Figure 7 shows the strategy for cDNA cloning.

Figure 8 shows the EcoRI-insert in the plasmid pDE2, named as pDE2-Fc$_\xi$R-1 vector.

Figure 8' shows the expression of Fc$_\xi$R cDNA in transfected Cos-7 cells. The transfected Cos-7 cells were stained with phycocyanin-conjugated anti-Fc$_\xi$R antibody (3-5) and biotinated IgE, developed with FITC-avidin and analyzed by a dual laser FACS; a) cells transfected with pDE2 containing human IFN-ß cDNA; b) cells transfected with pDE-2-Fc$_\xi$R-1. Contour plots represent the correlated expression of two surface determinants by showing peak lines enclosing equal percentage of cells with the two parameter distribution. X and Y axes represent the green and red log fluorescence intensities respectively.

Figure 9 shows the EcoRI-insert in the plasmid pGEM-4.

Figure 9' shows the expression of a Fc$_\xi$R cDNA in Xenopus oocyctes. Oocytes injected with mRNA transcript of pFc$_\xi$R-1, control mRNA or with mRNA from 8866 cells were incubated for 2 days and lysed, the levels of Fc$_\xi$R in lysates were measured by ELISA.

Table 1:       Purification of Fc$_\varepsilon$R from RPMI-8866 cells

| Material | Total Protein (µg) | Total Activity (units*) | Specific Activity units/µg | Percent Recovery | Purifi- cation |
|---|---|---|---|---|---|
| Cell culture Supernatant | 180.000 | 78.800 | 0.44 | 100 | 1 |
| Concentrated Supernatant (190X) | 172.000 | 75.000 | 0.44 | 95.2 | 1 |
| NMIg Effluent | 132.000 | 55.000 | 0.42 | 70 | 1 |
| 3-5-Seph. Eluate | 441 | 36.800 | 83.4 | 47 | 190 |
| HPLC | 16 | 26.000 | 1.630 | 33 | 3.710 |
| Crude Cell lysate | 117.000 | 6.160 | 0.05 | 100 | 1 |
| NMIg Effluent | 99.000 | 2.475 | 0.02 | 40.2 | 0.47 |
| 3-5-Seph. Eluate | 306 | 3.795 | 12.4 | 61.6 | 236 |
| HPLC- purified | — | 649 | 649 | 10.5 | 12.400 |

* 1 unit is the activity of 1 x $10^5$ cell equivalent of 190X concentrated culture supernatant.

Table 2

| Material | 3-5-Sepharose | | NMIg-Sepharose | | IgE-Sepharose | |
|---|---|---|---|---|---|---|
| | Eluate | Effluent | Eluate | Effluent | Eluate | Effluent |
| HPLC purfied | 2,470 | 4.5 | 85.5 | 1,170 | | |

## Table 3

```
       CTCCT GCTTAAACCT CTGTCTCTGA CGGTCCCTGC       35
       GAGGA CGAATTTGGA GACAGAGACT GCCAGGGACG

CAATCGCTCT GGTCGACCCC AACACACTAG GAGGACAGAC ACAGGCTCCA       85
GTTAGCGAGA CCAGCTGGGG TTGTGTGATC CTCCTGTCTG TGTCCGAGGT

AACTCCACTA AGTGACCAGA GCTGTGATTG TGCCCGCTGA GTGGACTGCG       135
TTGAGGTGAT TCACTGGTCT CGACACTAAC ACGGGCGACT CACCTGACGC

TTGTCAGGGA GTGAGTGCTC CATCATCGGG AGAATCCAAG CAGGACCGCC       185
AACAGTCCCT CACTCACGAG GTAGTAGCCC TCTTAGGTTC GTCCTGGCGG
```

```
                   5            10                15
Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG     230
TAC CTC CTT CCA GTT ATA AGT CTC TAG CTC CTC GAA GGG TCC TCC

                   20           25                30
Arg Cys Cys Arg Arg Gly Thr Gln Ile Val Leu Leu Gly Leu Val
CGG TGT TGC AGG CGT GGG ACT CAG ATC GTG CTG CTG GGG CTG GTG     275
GCC ACA ACG TCC GCA CCC TGA GTC TAG CAC GAC GAC CCC GAC CAC

                   35           40                45
Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG     320
TGG CGG CGA GAC ACC CGA CCC GAC GAC TGA GAC GAA GAG GAC ACC

                   50           55                60
His Trp Asp Thr Thr Gln Ser Leu Lys Gln Leu Glu Glu Arg Ala
CAC TGG GAC ACC ACA CAG AGT CTA AAA CAG CTG GAA GAG AGG GCT     365
GTG ACC CTG TGG TGT GTC TCA GAT TTT GTC GAC CTT CTC TCC CGA

                   65           70                75
Ala Arg Asn Val Ser Gln Val Ser Lys Asn Leu Glu Ser His His
GCC CGG AAC GTC TCT CAA GTT TCC AAG AAC TTG GAA AGC CAC CAC     410
CGG GCC TTG CAG AGA GTT CAA AGG TTC TTG AAC CTT TCG GTG GTG

                   80           85                90
Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG     455
CCA CTG GTC TAC CGC GTC TTT AGG GTC AGG TGC GTC TAA AGT GTC

                   95           100               105
Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG     500
CTT GAC CTC CTT GAA GCT CGA CTT GTC GTC TCT AAC TTT AGA GTC
```

```
              110                 115                 120
Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG      545
CTG AAC CTC GAC AGG ACC TTG GAC TTG CCC GAA GTT CGT CTA GAC

              125                 130                 135
Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA      590
TCG TCG AAG TTC AGG GTC CTT AAC TTG CTC TCC TTG CTT CGA AGT

              140                 145                 150
Asp Leu Leu Glu Arg Leu Arg Glu Glu Val Thr Lys Leu Arg Met
GAT TTG CTG GAA AGA CTC CGG GAG GAG GTG ACA AAG CTA AGG ATG      635
CTA AAC GAC CTT TCT GAG GCC CTC CTC CAC TGT TTC GAT TCC TAC

              155                 160                 165
Glu Leu Gln Val Ser Ser Gly Phe Val Cys Asn Thr Cys Pro Glu
GAG TTG CAG GTG TCC AGC GGC TTT GTG TGC AAC ACG TGC CCT GAA      680
CTC AAC GTC CAC AGG TCG CCG AAA CAC ACG TTG TGC ACG GGA CTT

              170                 175                 180
Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC      725
TTC ACC TAG TTA AAG GTT GCC TTC ACG ATG ATG AAG CCG TTC CCG

              185                 190                 195
Thr Lys Gln Trp Val His Ala Arg Tyr Ala Cys Asp Asp Met Glu
ACC AAG CAG TGG GTC CAC GCC CGG TAT GCC TGT GAC GAC ATG GAA      770
TGG TTC GTC ACC CAG GTG CGG GCC ATA CGG ACA CTG CTG TAC CTT

              200                 205                 210
Gly Gln Leu Val Ser Ile His Ser Pro Glu Glu Gln Asp Phe Leu
GGG CAG CTG GTC AGC ATC CAC AGC CCG GAG GAG CAG GAC TTC CTG      815
CCC GTC GAC CAG TCG TAG GTG TCG GGC CTC CTC GTC CTG AAG GAC

              215                 220                 225
Thr Lys His Ala Ser His Thr Gly Ser Trp Ile Gly Leu Arg Asn
ACC AAG CAT GCC AGC CAC ACC GGC TCC TGG ATT GGC CTT CGG AAC      860
TGG TTC GTA CGG TCG GTG TGG CCG AGG ACC TAA CCG GAA GCC TTG

              230                 235                 240
Leu Asp Leu Lys Gly Glu Phe Ile Trp Val Asp Gly Ser His Val
TTG GAC CTG AAG GGA GAG TTT ATC TGG GTG GAT GGG AGC CAT GTG      905
AAC CTG GAC TTC CCT CTC AAA TAG ACC CAC CTA CCC TCG GTA CAC

              245                 250                 255
Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG      950
CTG ATG TCG TTG ACC CGA GGT CCC CTC GGG TGG TCG GCC TCG GTC

              260                 265                 270
Gly Glu Asp Cys Val Met Met Arg Gly Ser Gly Arg Trp Asn Asp
GGC GAG GAC TGC GTG ATG ATG CGG GGC TCC GGT CGC TGG AAC GAC      995
CCG CTC CTG ACG CAC TAC TAC GCC CCG AGG CCA GCG ACC TTG CTG
```

```
                     275                        280                        285
          Ala Phe Cys Asp Arg Lys Leu Gly Ala Trp Val Cys Asp Arg Leu
          GCC TTC TGC GAC CGT AAG CTG GGC GCC TGG GTG TGC GAC CGG CTG 1040
          CGG AAG ACG CTG GCA TTC GAC CCG CGG ACC CAC ACG CTG GCC GAC

                                280                        295                        300
          Ala Thr Cys Thr Pro Pro Ala Ser Glu Gly Ser Ala Glu Ser Met
          GCC ACA TGC ACG CCG CCA GCC AGC GAA GGT TCC GCG GAG TCC ATG 1085
          CGG TGT ACG TGC GGC GGT CGG TCG CTT CCA AGG CGC TCC AGG TAC

                                305                        310                        315
          Gly Pro Asp Ser Arg Pro Asp Pro Asp Gly Arg Leu Pro Thr Pro
          GGA CCT GAT TCA AGA CCA GAC CCT GAC GGC CGC CTG CCC ACC CCC 1130
          CCT GGA CTA AGT TCT GGT CTG GGA CTG CCG GCG GAC GGG TGG GGG

          Ser Ala Pro Leu His Ser  *
          TCT GCC CCT CTC CAC TCT TGA GCATGGATA CAGCCAGGCC CAGAGCAAGA 1180
          AGA CGG GGA GAG GTG AGA ACT CGTACCTAT GTCGGTCCGG GTCTCGTTCT

          CCCTGAAGAC CCCCAACCAC GGCCTAAAAG CCTCTTTGTG GCTGAAAGGT            1230
          GGGACTTCTG GGGGTTGGTG CCGGATTTTC GGAGAAACAC CGACTTTCCA

          CCCTGTGACA TTTTCTGCCA CCCAAACGGA GGCAGCTGAC ACATCTCCCG            1280
          GGGACACTGT AAAAGACGGT GGGTTTGCCT CCGTCGACTG TGTAGAGGGC

          CTCCTCTATG GCCCCTGCCT TCCCAGGAGT ACACCCCAAC AGCACCCTCT            1330
          GAGGAGATAC CGGGGACGGA AGGGTCCTCA TGTGGGGTTG TCGTGGGAGA

          CCAGATGGGA GTGCCCCCAA CAGCACCCTC TCCAGATGAG AGTACACCCC            1380
          GGTCTACCCT CACGGGGGTT GTCGTGGGAG AGGTCTACTC TCATGTGGGG

          AACAGCACCC TCTCCAGATG CAGCCCCATC TCCTCAGCAC CCCAGGACCT            1430
          TTGTCGTGGG AGAGGTCTAC GTCGGGGTAG AGGAGTCGTG GGGTCCTGGA

          GAGTATCCCC AGCTCAGGTG GTGAGTCCTC CTGTCCAGCC TGCATCAATA            1480
          CTCATAGGGG TCGAGTCCAC CACTCAGGAG GACAGGTCGG ACGTAGTTAT

          AAATGGGGCA GTGATGGCCT CCCA                                       1504
          TTTACCCCGT CACTACCGGA GGGT
```

We claim:

1. Human low affinity Fcζ-receptor with a N-terminal cytoplasmic domain, a C-terminal extracellular domain and a molecular weight of about 46 kd and the glycosylated derivate thereof.

2. Human low affinity Fcζ-receptor as claimed in claim 1 containing the following partial amino acid sequences:

Met-Glu-Leu-Gln-Val-Ser-Ser-Gly-Phe-Val-,

Gly-Glu-Phe-Ile-Trp-Val-Asp-Gly-Ser-His-Val-Asp-Tyr-Ser-Asn-Trp-Ala-Pro-Gly-Glu-Pro-Thr-,

Lys-His-Ala-Ser-His-Thr-Gly-Ser-Trp-Ile-Gly-Leu-Arg-Asn-Leu-Asp-Leu-Lys- and

Lys-Trp-Ile-Asn-Phe-Gln-.

3. Human low affinity Fcζ-receptor as claimed in claim 1 containing the partial amino acid sequence of the formula

Lys-Trp-Ile-Asn-Phe-Gln-.

4. Human low affinity Fcζ-receptor as claimed in any of the claims 1 to 3 showing the formula

```
Met Glu Glu Gly Gln Tyr Ser Glu Ile Glu Glu Leu Pro Arg Arg
Arg Cys Cys Arg Arg Gly Thr Gln Ile Val Leu Leu Gly Leu Val
Thr Ala Ala Leu Trp Ala Gly Leu Leu Thr Leu Leu Leu Leu Trp
His Trp Asp Thr Thr Gln Ser Leu Lys Gln Leu Glu Glu Arg Ala
Ala Arg Asn Val Ser Gln Val Ser Lys Asn Leu Glu Ser His His
Gly Asp Gln Met Ala Gln Lys Ser Gln Ser Thr Gln Ile Ser Gln
Glu Leu Glu Glu Leu Arg Ala Glu Gln Gln Arg Leu Lys Ser Gln
Asp Leu Glu Leu Ser Trp Asn Leu Asn Gly Leu Gln Ala Asp Leu
Ser Ser Phe Lys Ser Gln Glu Leu Asn Glu Arg Asn Glu Ala Ser
Asp Leu Leu Glu Arg Leu Arg Glu Glu Val Thr Lys Leu Arg Met
Glu Leu Gln Val Ser Ser Gly Phe Val Cys Asn Thr Cys Pro Glu
```

Lys Trp Ile Asn Phe Gln Arg Lys Cys Tyr Tyr Phe Gly Lys Gly
Thr Lys Gln Trp Val His Ala Arg Tyr Ala Cys Asp Asp Met Glu
Gly Gln Leu Val Ser Ile His Ser Pro Glu Glu Gln Asp Phe Leu
Thr Lys His Ala Ser His Thr Gly Ser Trp Ile Gly Leu Arg Asn
Leu Asp Leu Lys Gly Glu Phe Ile Trp Val Asp Gly Ser His Val
Asp Tyr Ser Asn Trp Ala Pro Gly Glu Pro Thr Ser Arg Ser Gln
Gly Glu Asp Cys Val Met Met Arg Gly Ser Gly Arg Trp Asn Asp
Ala Phe Cys Asp Arg Lys Leu Gly Ala Trp Val Cys Asp Arg Leu
Ala Thr Cys Thr Pro Pro Ala Ser Glu Gly Ser Ala Glu Ser Met
Gly Pro Asp Ser Arg Pro Asp Pro Asp Gly Arg Leu Pro Thr Pro
Ser Ala Pro Leu His Ser

5. Water-soluble part of human low affinity Fc$_\varepsilon$-receptor as claimed in any of the claims 1 to 4 starting with the amino acids from about 50 to about 150 of the amino acid sequence as claimed in claim 4 and the glycosylated derivate thereof.

6. Water-soluble part of human low affinity Fc$_\varepsilon$-receptor as claimed in claim 5 with a molecular weight of about 25 kd.

7. Water-soluble part of human low affinity Fc$_\varepsilon$-receptor as claimed in claim 5 or 6 containing the amino acid sequence of formula

```
                                                      Met
Glu Leu Gln Val Ser Ser Gly Phe Val Cys Asn Thr Cys Pro Glu
Lys Trp Ile Asn Phe Gln Arg Lys Cys Tyr Tyr Phe Gly Lys Gly
Thr Lys Gln Trp Val His Ala Arg Tyr Ala Cys Asp Asp Met Glu
Gly Gln Leu Val Ser Ile His Ser Pro Glu Glu Gln Asp Phe Leu
Thr Lys His Ala Ser His Thr Gly Ser Trp Ile Gly Leu Arg Asn
Leu Asp Leu Lys Gly Glu Phe Ile Trp Val Asp Gly Ser His Val
Asp Tyr Ser Asn Trp Ala Pro Gly Glu Pro Thr Ser Arg Ser Gln
Gly Glu Asp Cys Val Met Met Arg Gly Ser Gly Arg Trp Asn Asp
Ala Phe Cys Asp Arg Lys Leu Gly Ala Trp Val Cys Asp Arg Leu
Ala Thr Cys Thr Pro Pro Ala Ser Glu Gly Ser Ala Glu Ser Met
Gly Pro Asp Ser Arg Pro Asp Pro Asp Gly Arg Leu Pro Thr Pro
Ser Ala Pro Leu His Ser
```

8. Water-soluble part of human low affinity Fc$_\varepsilon$-receptor as claimed in any of the claims 5 to 7 showing the formula as claimed in claim 7.

9. Recombinant human low affinity Fc$_\varepsilon$-receptor as claimed in any of the claims 1 to 8 essentially free of other proteins of human origin.

10. Recombinant human low affinity Fcξ-receptor as claimed in claim 9 unaccompanied by associated native glycosylation.

11. Recombinant human low affinity Fcξ-receptor as claimed in the claims 9 and 10 produced by expression of the recombinant DNA-sequence of formula

```
ATG GAG GAA GGT CAA TAT TCA GAG ATC GAG GAG CTT CCC AGG AGG
TAC CTC CTT CCA GTT ATA AGT CTC TAG CTC CTC GAA GGG TCC TCC

CGG TGT TGC AGG CGT GGG ACT CAG ATC GTG CTG CTG GGG CTG GTG
GCC ACA ACG TCC GCA CCC TGA GTC TAG CAC GAC GAC CCC GAC CAC

ACC GCC GCT CTG TGG GCT GGG CTG CTG ACT CTG CTT CTC CTG TGG
TGG CGG CGA GAC ACC CGA CCC GAC GAC TGA GAC GAA GAG GAC ACC

CAC TGG GAC ACC ACA CAG AGT CTA AAA CAG CTG GAA GAG AGG GCT
GTG ACC CTG TGG TGT GTC TCA GAT TTT GTC GAC CTT CTC TCC CGA

GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC

AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT

GAT TTG CTG GAA AGA CTC CGG GAG GAG GTG ACA AAG CTA AGG ATG
CTA AAC GAC CTT TCT GAG GCC CTC CTC CAC TGT TTC GAT CCC TAC

GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

GAG CAG CTG GTC AGC ATC CAC AGC CCG GAG GAG CAG GAC TTC CTG
CTC GTC GAC CAG TCG TAG GTG TCG GGC CTC CTC GTC CTG AAG GAC

AAG AAG CAT GCC AGC CAC ACC GGC TCC TGG ATT GGC CTT CGG AAC
TTC TTC GTA CGG TCG GTG TGG CCG AGG ACC TAA CCG GAA GCC TTG
```

```
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

TCT GCC CCT CTC CAC TCT TGA
AGA CGG GGA GAG GTG AGA ACT
```

or of a degenerative derivate thereof.

12. Water-soluble part of recombinant human lower affinity Fc$_\epsilon$-receptor as claimed in claims 9 and 10 produced by expression of a recombinant DNA-sequence starting with the codons from about 50 to about 150 of the DNA-sequence as claimed in claim 11.

13. Water-soluble part of recombinant human lower affinity Fc$_\epsilon$-receptor as claimed in claims 9 and 10 produced by expression of a recombinat DNA-sequence containing at least the sequence of formula

```
                                                            ATG
                                                            TAC

GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC
```

```
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

GCC ACA TGC ACG CCG CCA GCC AGC GAA GGT TCC GCG GAG TCC ATG
CGG TGT ACG TGC GGC GGT CGG TCG CTT CCA GGC GCC CTC AGG TAC

GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

TCT GCC CCT CTC CAC TCT TGA
AGA CGG GGA GAG GTG AGA ACT
```

or of a degenerative derivate thereof.

14. Water soluble part of human lower affinity $Fc_\varepsilon$-receptor as claimed in claims 9 and 10 produced by expression of the recombinant DNA-sequence as claimed in claim 13.

15. A recombinant DNA molecule which contains the genetic information as claimed in any of the claims 1 to 14 or a degenerative derivate thereof.

16. A recombinant DNA molecule as claimed in claim 15 which is a plasmid vector, phage vector or cosmid vector.

17. The plasmid of claim 15 wherein the coding sequence is as claimed in any of the claims 11 to 14.

18. The plasmid LE392 as claimed in claim 15 deposited in E.coli HB 101 under number FERM BP-1116 containing in the plasmid pGEM$^{TM}$4 the DNA-sequence as claimed in claim 11 as EcoRI-insert.

19. A recombinant DNA molecule as claimed in claim 15 containing the replicon and control sequences for expression.

20. A recombinant DNA molecule as claimed in claim 19 which is an expression vector suitable for transformation of E. coli.

21. A recombinant DNA molecule as claimed in claim 20 containing the encoding sequence as claimed in claims 11 to 14 as EcoRI-insert.

22. A recombinant DNA molecule as claimed in claim 21, wherein as plasmid the plasmid pDE2 or pGEM-4 is used.

23. A recombinant DNA molecule as claimed in claim 19 which is a yeast expression vector.

24. A host organism transformed by a vector as claimed in any of the claims 16 to 23.

25. An oligonucleotide encoding for a partial amino acid sequence as claimed in claim 2 or 3.

26. The oligonucleotide as claimed in claim 25 showing the formula

$$3'-TT^T_C \ ACC \ TA\overset{T}{\underset{A}{G}} \ TT^A_G \ AA^A_G \ GT \ -5'$$

, which encodes for a part of the amino acid sequence as claimed in claim 3.

27. A process for preparing a polypeptide as claimed in any one of claims 1 to 8 which comprises transforming a suitable host organism with an expression vector containing a coding sequence as claimed in any of the claims 9 to 14 for the desired polypeptide at an appropriate site for expression and isolating the desired polypeptide from the resulting transformants.

28. Process for the preparation of human low affinity
Fc$_\xi$-receptor as claimed in any of the claims 5 to 8 which
comprises

culturing B lymphoblastoid cells and separating said Fc$_\xi$R
from the supernatant or from the lysed cells by sequential
immunoaffinity purification.

29. Process as claimed in claim 28, wherein RPMI-8866 cells
are used as lymphoblastoid cells.

30. Process as claimed in claims 28 or 29, wherein a solu-
tion containing Fc$_\xi$R is sequentially absorbed on BSA-Sepharose, human transferrin Sepharose and normal mouse Ig-Sepharose, the effluent is applied to an anti-Fc$_\xi$R-affinity co-
lumn and the eluent is further purified by means of HPLC.

31. Process as claimed in claim 30, wherein the immunoaffinity column is prepared by coupling a corresponding antibody
to Sepharose.

32. Process for the preparation of partial amino acid sequences as claimed in claims 2 or 3 wherein Fc$_\xi$-receptor as
prepared according to claim 28 is subjected to trypsin or
lysylendopeptidase treatment and subsequently separated by
means of HPLC.

33. Process as claimed in claim 32, wherein the partial
amino acid sequence as claimed in claim 3 is isolated.

34. Process for the preparation of the DNA-sequence as claimed in claim 25 or 26 wherein the oligonucleotide is synthesized by addition of the corresponding nucleotides by means
of an oligo synthesizer.

35. A process for identifying expression vehicles containing genes coding for $Fc_\xi R$ as claimed in claims 1 to 8, comprising the steps of:

synthesizing cDNA from an RNA matrix derived from lymphoblastoid cells producing $Fc_\xi R$ mRNA,

incorporating said synthesized cDNA in expression vehicles to form an expression vehicle bank,

hybridizing said incorporated cDNA to identify those expression vehicles which contain a gene coding for $Fc_\xi R$, with two labelled probes comprising cDNA specific to $Fc_\xi R^+ L$ cell and an oligonucleotide common to gene of $Fc_\xi R$.

36. Process as claimed in claim 35, wherein said probe I is synthesized from a matrix of RNA, which was isolated from $Fc_\xi R^+ L$ cells.

37. Process as claimed in claim 35, wherein said probe II is synthesized from a matrix of RNA containing $Fc_\xi R$ sequences and said oligonucleotide as claimed in claim 25 or 26 is a primer.

38. Process as claimed in claim 35, wherein said lymphoblastoid cell are cells of line RPMI-8866.

39. Process as claimed in claim 35, wherein said $Fc_\xi R^+ L$ cells are obtained by co-transfection of $Ltk^-$ cells with Herpes simplex virus derived tk gene and high molecules genomic DNA from RPMI-8866 cells.

40. Process for preparing a host organism as claimd in claim 24, wherein a vector as claimed in claims 15 to 23 is transformed with a suitable host.

41. Process for preparing a vector as claimed in claims 15 to 23, wherein a DNA-sequence as claimed in claims 11 to 15 is inserted in a suitable vector.

42. Pharmaceutical compositions containing a polypeptide as claimed in claim 1 to 8.

43. Pharmaceutical compositions as claimed in claim 42 suitable for treatment of local or systhemic IgE-allergic reactions.

44. Preparation of a pharmaceutical composition as claimed in claims 42 or 43 wherein an effective amount of a polypeptide as claimed in any of the claims 1 to 8 is incorporated in one or more excipients.

45. Use of a polypeptide as claimed in any of the claims 1 to 8 for the preparation of a pharmaceutical composition.

Boehringer Ingelheim Zentrale GmbH · D-6507 Ingelheim am Rhein

Europäisches Patentamt
Erhardtstraße 27

8000 München 2

Boehringer Ingelheim
Zentrale GmbH
Zentral-Abteilung Patente

| Ihr Zeichen | Ihre Nachricht vom | Unser Zeichen | Telefon-Durchwahl | Ingelheim am Rhein, |
|---|---|---|---|---|
| | | Rm/sch | 0 61 32 - 77 - | den September 29, 1986 |

European Patent Application 86 113 073.0, Case 12/036IP-Mi/Fl
Tadamitsu Kishimoto

Under the provisions of Rule 88 we hereby request permission
to correct the applicant's address. We enclose in triplicate
amended page 1 of the Request for Grant.

We would also like to take this opportunity of requesting the
following amendment to the specification at page 7, lines 6/7
by deleting the words "The first .... and therefore".

The word "two" should then subsequently read "Two".

Three copies of the amended page are filed herewith.

Very truly yours,

R. Milnes

Enclosure
Page 1 of the Request for Grant (in triplicate)

☎ 0 61 32 - 77 - 0
418 791 - 0 bi d
Boehringer Ingelheimrhein
Fax 0 61 32 - 77 - 3000/3080/3940

Zentral-Geschäftsleitung:
Hubertus Liebrecht (Vorsitzender),
Dr. Folkert Bellstedt, Erich von Baumbach,
Herbert W. Grimm, Prof. Dr. Franz Waldeck

Vorsitzender des Aufsichtsrates:
Prosper Graf zu Castell-Castell
Sitz Ingelheim am Rhein
Registergericht Bingen – HR B 1005